# DEMANDE DE BREVET EUROPEEN

(11) **EP 2 168 525 A1**
(43) Date de publication de la demande: **31.03.2010**
(21) Numéro de dépôt: 08370020.3
(22) Date de dépôt: 25.09.2008
(51) Int. Cl.: A61B 19/00, A61B 18/24

(54) **Appareil de traitement comportant un instrument invasif et des moyens d'aide au controle du déplacement dudit instrument**

(71) Demandeur: Osyris Medical, 59260 Hellemmes Lille (FR)
(72) Inventeur: Zemmouri, Jaouad, 59242 Genech (FR); Rochon, Phillipe, 59182 Loffre (FR); Lefevre, Liliane, 59000 Lille (FR)
(74) Mandataire: Matkowska, Franck

(57) **Abrégé**

L'appareil de traitement comportant un instrument invasif (par exemple un ensemble canule/fibre optique(12) ou cathéter/fibre optique) qui est apte a être introduit au moins partiellement dans une partie de corps humain ou animal, et par exemple dans tine veine, et qui permet de délivrer localement des doses de traitement (par exemple des doses d'énergie électromagnétique) dans ladite partie de corps humain ou animal. Cet appareil comporte des moyens (2) d'aide au contrôle du déplacement de l'instrument dans la partie de corps humain ou animal, lesquels moyens d'aide au contrôle de déplacement comportent au moins un repère visuel (RL), par exemple un repère lumineux, et des moyens de commande permettant de déplacer automatiquement et linéairement ledit au moins repère visuel (RL), à une vitesse prédéfinie.

## Description

### Domaine technique

La présente invention concerne le domaine du traitement du corps humain ou animal au moyen d'un instrument invasif, qui est apte à être introduit au moins partiellement dans une partie de corps humain ou animal, de manière à délivrer localement des doses de traitement à l'intérieur de la partie de corps humain ou animal. L'invention trouve principalement, mais non exclusivement, son application dans le domaine des traitements endoveineux, par irradiation électromagnétique ou radiofréquence (RF), ou par injection de vapeur d'eau.

### Art antérieur

Dans le domaine des traitements thérapeutiques, prophylactiques ou cosmétiques du corps humain ou animal, au moyen d'un instrument invasif qui est utilisé pour délivrer localement des doses de traitement à l'intérieur de la partie de corps humain ou animal, la dosimétrie est un élément important qui conditionne l'efficacité du traitement.

Plus particulièrement, dans le domaine des traitements endoveineux par exemple par irradiation électromagnétique, on utilise comme instrument invasif un ensemble canule/fibre optique ou cathéter/fibre optique ; la fibre optique est reliée à une source laser, et la canule permet l'insertion et le guidage de l'extrémité distale libre la fibre optique à l'intérieur de la veine à traiter, c'est-à-dire l'extrémité de la fibre optique par laquelle le rayonnement électromagnétique est émis dans la veine.

Différentes études cliniques ont montré que l'efficacité du laser endoveineux était dépendante de l'application d'une dose (généralement exprimée en LEED: *Linear* Endovenous *Energy Density*) en accord avec le diamètre de la veine mesurée en position orthostatique. Dans cette application, la dosimétrie correspond à la quantité d'énergie E(J) délivrée en tout point de la veine traitée : E(J)= Puissance laser(W) x Tps d'exposition du point(s).Cette quantité d'énergie déposée a un impact directement sur l'effet tissulaire (élévation de température, nécrose, ...). Une dosimétrie insuffisante conduit à une fermeture partielle de la veine, pouvant conduire à une revascularisation, partielle ou totale. Une dosimétrie trop élevée peut conduire à l'apparition de brûlures de tissus voisins.

Dans le domaine des traitements endoveineux, une fois l'extrémité d'émission de la fibre optique introduite dans la veine, le praticien effectue des tirs laser en tirant manuellement sur la fibre optique, de manière à retirer progressivement la fibre optique de la veine. Il existe principalement deux techniques de retrait de la fibre optique.

Selon une première technique, dite de retrait point par point, le praticien positionne de manière discontinue l'extrémité d'émission de la fibre optique à différents points de la veine, et effectue un tir laser (pulsé ou continu) à chaque position de la fibre optique. Avec cette technique de retrait point par point, le temps d'exposition est parfaitement défini et correspond à la durée du ou des pulses laser. Le contrôle de la dose délivrée en chaque point est donc avantageusement facilité. En revanche, la pratique du tir point par point est relativement contraignante pour le praticien.

Selon une deuxième technique, dite de retrait continu, le praticien procède au retrait continu de la fibre optique, et simultanément à ce mouvement de retrait continu actionne la source d'énergie (laser). Cette deuxième technique présente l'avantage pour le praticien d'être moins contraignante et plus simple, et permet une mise en oeuvre plus rapide du traitement, comparativement à la technique de retrait point par point. En revanche, en mode de retrait continu, le temps d'exposition, et de ce fait la dose délivrée, dépendent de la vitesse de retrait de la fibre. Cette vitesse de retrait étant dépendante du praticien, il en résulte qu'il est difficile en pratique d'obtenir un déplacement régulier continu de la fibre avec par conséquent, de nombreux surdosages ou sous dosages possibles lors du traitement.

Afin d'aider le praticien à appliquer une dosimétrie correcte lors de la procédure de retrait continu, un dispositif d'assistance tire-fibre est apparu récemment sur le marché. Ce dispositif tire-fibre est conçu pour tirer automatiquement la fibre optique à une vitesse prédéterminée, à la place du praticien. Sur ce dispositif, seul le mode de retrait continu est disponible. L'expérience montre toutefois que ce dispositif tire-fibre « patine » parfois, ce qui peut provoquer accidentellement un surdosage. En outre sa mise en oeuvre n'est pas simple et ne prend pas en compte d'éventuels mouvements du patient.

### Objectif de l'invention

Un objectif de l'invention est de proposer une nouvelle solution technique permettant d'aider un praticien lors d'un traitement invasif, et notamment lors d'un traitement endoveineux, à délivrer de façon reproductible et précise les doses de traitement qui ont été prédéfinies, en particulier lorsque le praticien délivre les doses de traitement en effectuant un retrait continu de l'instrument de délivrance des doses.

### Résumé de l'invention

Cet objectif est atteint par l'appareil de traitement de la revendication 1. Cet appareil comporte un instrument invasif qui est apte à être introduit au moins partiellement dans une partie de corps humain ou animal, et qui permet de délivrer localement des doses de traitement dans ladite partie de corps humain ou animal, et des moyens d'aide au contrôle du déplacement de l'instrument dans la partie de corps humain ou animal. Ces moyens d'aide au contrôle de déplacement comportent au moins un repère visuel et des moyens de commande permettant de déplacer automatiquement et linéairement ledit au moins repère visuel à une vitesse prédéfinie.

Ainsi, grâce à l'invention, le praticien dispose au cours du traitement d'un repère visuel qui se déplace à une vitesse prédéfinie, et qui constitue avantageusement une référence de vitesse. Le praticien peur ainsi très facilement contrôler son geste de retrait continu de l'instrument, et par exemple de retrait continu de la fibre optique, en suivant le déplacement ce repère visuel, ce qui lui permet de contrôler facilement la vitesse de retrait de l'instrument, et par conséquent la dosimétrie du traitement.

L'invention a pour autre objet un procédé de traitement d'une partie de corps humain ou animal, qui est **caractérisé en ce qu**'on introduit au moins partiellement, dans la partie de corps humain ou animal à traiter, un instrument permettant de délivrer localement une dose de traitement qui dépend de la vitesse de déplacement de l'instrument, et on délivre une dose de traitement au moyen de cette instrument en procédant au retrait continu de l'instrument, et en ce que pour procéder à ce retrait continu de l'instrument , on suit manuellement le déplacement d'un repère visuel qui se déplace linéairement et automatiquement à une vitesse prédéfinie.

Plus particulièrement, le repère visuel peut être un repère lumineux.

Dans une variante de mise en oeuvre, on délivre une dose en effectuant un tir laser (continu ou pulsé) au cours du mouvement continu de retrait de l'instrument.

Plus particulièrement, le procédé est un traitement endoveineux au cours duquel l'instrument est introduit dans une veine.

L'invention a également pour objet l'utilisation de l'appareil visé précédemment pour réaliser un traitement endoveineux en mode de retrait continu.

### Brève description des figures

D'autres caractéristiques de l'invention apparaîtront plus clairement à la lecture de la description détaillée ci-après de plusieurs variantes de réalisation de l'invention, laquelle description est donnée à titre d'exemple non limitatif et non exhaustif de l'invention, en référence aux dessins annexés sur lesquels :
- la figure 1 est une représentation schématique d'un laser endoveineux,
- la figure 2 est une représentation en perspective d'un exemple de règle lumineuse de l'invention,
- la figure 3 est un schéma bloc du circuit de commande de la règle lumineuse de la figure 2,
- la figure 4 représente de manière schématique une règle lumineuse avec un repère lumineux unique (diode allumée) et la fibre optique du laser endoveineux de la figure 1,
- la figure 5 représente de manière schématique une règle lumineuse avec plusieurs repères lumineux (diodes allumées).

### Description détaillée

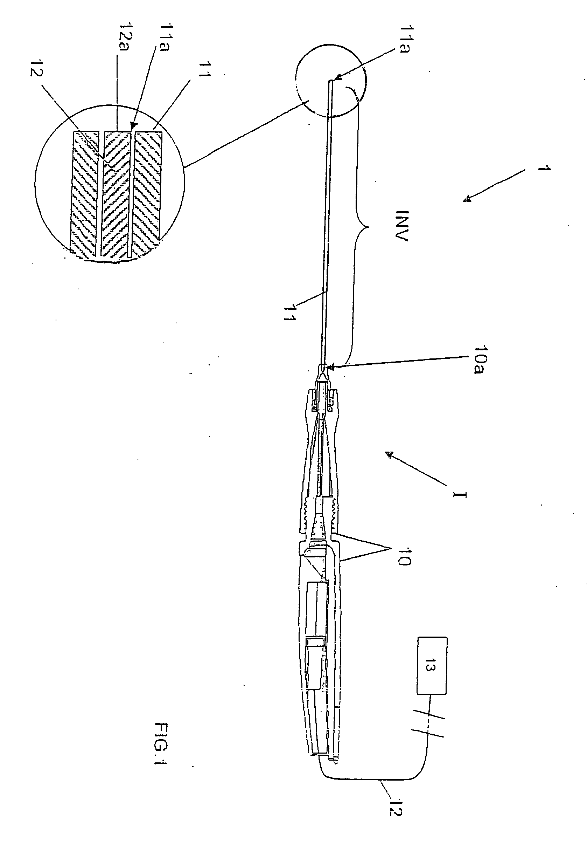

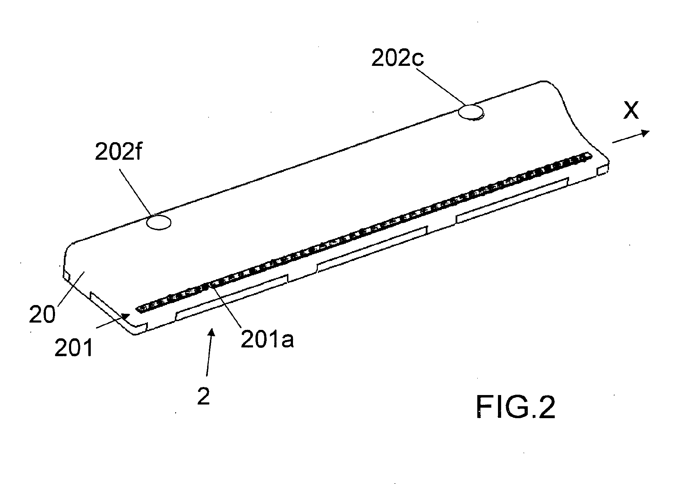

On a représenté sur les figures 1 et 2, les deux principaux composants d'un appareil de traitement de l'invention. Dans cet exemple particulier, mais de manière non limitative et non exhaustive de l'invention cet appareil de traitement comporte un laser endoveineux 1 (figure 1), qui est connu en soi, et un dispositif 2 (figure 2) qui permet de fournir au praticien une référence de vitesse, et qui est spécifique de l'invention.

### Laser endoveineux (1)

De manière connue, le laser endoveineux 1 comporte un instrument de traitement invasif I comprenant une pièce à main 10 sur laquelle est fixée une canule 11, et une fibre optique 12 qui est enfilée dans la pièce à main 10 et dans la canule 11 et qui est immobilisée par rapport à ladite canule11. Dans une autre variante, l'ensemble canule 11/fibre optique 12 du laser endoveineux peut être remplacé par un ensemble cathéter/fibre optique ou par une fibre optique uniquement.

L'extrémité distale 12a de la fibre optique constitue la sortie de l'instrument I par laquelle les doses de traitement sont délivrées. Dans l'exemple particulier illustré sur de la figure 1, l'extrémité distale 12a de la fibre optique 12 affleure avec l'ouverture distale 11a de la canule. Dans une autre variante, l'extrémité distale 12a de la fibre optique 12 pourrait être située à l'extérieur de la canule 11, mais à proximité immédiate de l'ouverture distale 11a de la canule 11.

La fibre optique 12 est reliée à son autre extrémité à un générateur de rayonnement électromagnétique 13. La fréquence d'émission du générateur 13 sera choisie de manière connue en soi par l'homme du métier, et peut selon le type de traitement être dans le domaine du visible, de l'infrarouge, des hyperfréquences ou radiofréquences. La fréquence d'émission et/ou la puissance de du générateur de rayonnement électromagnétique sont de préférence réglables. Lorsque le générateur 13 de rayonnement électromagnétique fonctionne, l'instrument I délivre à son extrémité distale d'émission 12a un rayonnement électromagnétique pouvant être appliqué à une partie du corps à traiter.

La pièce à main 10 permet la manipulation à la main de l'ensemble canule 11/fibre optique 12, et constitue une partie non invasive de l'instrument 1. La partie de l'ensemble canule11/fibre optique 12, qui est référencée « INV », qui est externe à la pièce à main 10, et qui s'étend depuis l'extrémité distale 10a de la pièce à main 10, constitue une partie invasive de l'instrument I destinée à être introduite partiellement ou totalement dans la partie du corps humain à traiter, et notamment dans la veine à traiter.

### Dispositif (2)

Le dispositif 2 comprend un boîtier 20 qui forme une règle lumineuse et qui est destiné à être posé sur la partie de corps humain à traiter, et par exemple sur la jambe d'un patient où est localisée la veine à traiter.

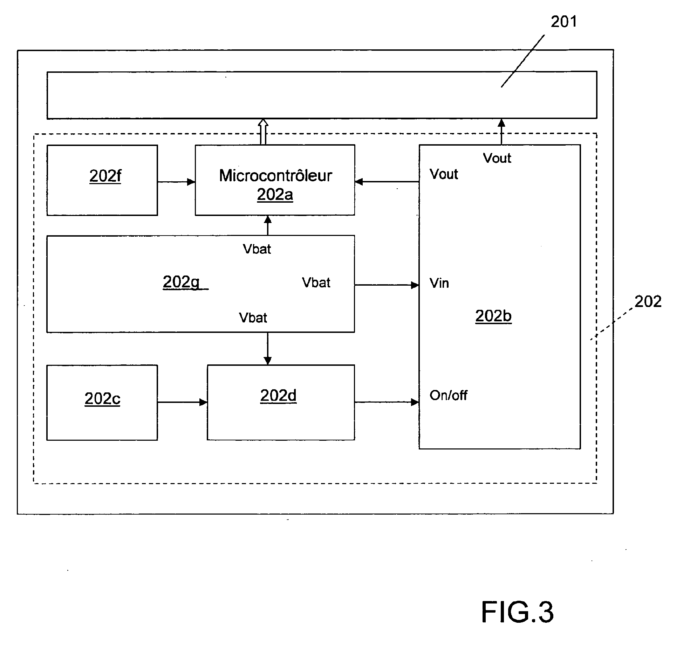

Ce boitier 20 comporte un ensemble 201 de diodes électroluminescentes 201a, qui sont alignées suivant un axe (X), et sert de logement à un circuit électronique 202 (figure 3) permettant de commander les diodes 201 a.

Un exemple de synoptique pour le circuit 202 est représenté sur la figure 3. Ce circuit électronique 202 comporte principalement :
- un microcontrôleur 202a permettant de commander l'allumage et l'extinction séquentiels, et en boucle, des diodes 201 à une vitesse prédéfinie,
- une source de tension continue 202b pour l'alimentation électrique des diodes 201 a,
- un bouton 202c March/Arrêt qui est associé à un module électronique 202d de gestion de ce bouton, et permet d'allumer et d'éteindre la règle lumineuse 20,
- un bouton 202f de sélection de vitesse, qui permet au praticien de sectionner la vitesse d'allumage des diodes 201 a parmi un ensemble de vitesses prédéfinies,
- une batterie 202g permettant d'alimenter électriquement (tension Vbat) le microcontrôleur 202a, la source de tension continue 202b, l'ensemble 201 de diodes 201a, et le module électronique 202d de gestion du bouton March/Arrêt 202c.

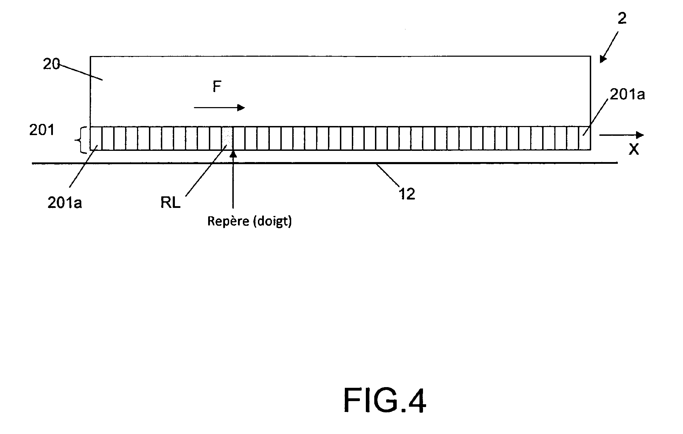

Le microcontrôleur 202a est programmé pour allumer et éteindre séquentiellement chaque diode 201 a l'une après l'autre, et en boucle, à une vitesse constante prédéfinie correspondant à la vitesse qui a été sélectionnée par le praticien au moyen du bouton de sélection 202f. Ainsi, cette commande séquentielle des diodes 201 a permet de fournir au praticien un repère visuel lumineux RL (figure 4 - diode couramment allumée) qui se déplace linéairement suivant l'axe (X) d'alignement des diodes (figure 4- flèche F) à ladite vitesse prédéfinie d'allumage et d'extinction des diodes. La séquence d'allumage et d'extinction séquentielle des diodes 201 a est réalisée en boucle par le microcontrôleur 202a, de telle sorte qu'une fois la dernière diode allumée, puis éteinte, le microcontrôleur redémarre automatiquement une séquence à partir de la première diode opposée.

Dans une variante de réalisation, le sens de déplacement de ce repère visuel lumineux RL suivant l'axe (X) d'alignement des diodes 201a (c'est-à-dire l'ordre d'allumage et d'extinction des diodes) peut avantageusement, mais non nécessairement, être inversé, par exemple par un appuie prolongé sur le bouton de sélection de vitesse 202f.

Plus particulièrement, la vitesse sélectionnée et le cas échéant le sens de défilement du repère lumineux (RL) sont de préférence mémorisés, de telle sorte que lorsque la règle 20 est arrêtée, puis remise en marche au moyen du bouton 202c, la vitesse d'allumage et d'extinction des diodes et le sens de défilement du repère lumineux (RL) sont par défaut ceux enregistrés en mémoire, ce qui évite le cas échéant au praticien de devoir paramétrer de nouveau la règle 20, lorsque ces deux paramètres (vitesse et sens de défilement) ne doivent pas être modifiés.

### Exemple de mise en oeuvre de l'appareil de traitement

Le praticien positionne la règle lumineuse 20 sur le patient au niveau de la partie de corps humain à traiter, et par exemple sur la jambe d'un patient où est localisée la veine à traiter.

Le praticien met en marche la règle lumineuse 20 au moyen du bouton Marche/Arrêt 202c, et sélectionne le cas échéant une vitesse au moyen du bouton de sélection 202f ou conserve la vitesse enregistrée en mémoire.

Pour faciliter le choix de la vitesse, on peut fournir au praticien avec l'appareil de traitement une table donnant la puissance (W) du laser (mode continu) en fonction de la dose appliquée (J/cm) et de la vitesse de retrait. Un exemple de table est donné ci-après avec cinq vitesses différentes n°1 à n°5 et pour des doses de 40 J/cm, 50 J/cm, 60 J/cm, 70 J/cm et 80 J/cm.

| | Dose (J/cm) | | | | |
|---|---|---|---|---|---|
| Vitesse (n°) | **40** | **50** | **60** | **70** | **80** |
| **1** | 5,7 | 7,1 | 8,6 | 10,0 | 11,4 |
| **2** | 6,7 | 8,3 | 10,0 | 11,7 | 13,3 |
| **3** | 8,0 | 10,0 | 12,0 | 14,0 | |
| **4** | 10,0 | 12,5 | 15,0 | | |
| **5** | 12,0 | 15,0 | | | |

Le praticien choisit une vitesse (par exemple vitesse n°2) et une dose souhaitée (par exemple 60 J/cm), et règle la puissance du laser correspondante (par exemple 10W) ou pour une puissance donnée du laser (par exemple 10W) et une dose souhaitée (par exemple 60 J/cm), choisit la vitesse correspondante (par exemple vitesse n°2). Dans les deux cas, le praticien sélectionne la vitesse requise sur la règle lumineuse 20 au moyen du bouton de sélection 202f.

La vitesse sélectionnée est affichée sur la règle 20 par exemple en allumant temporairement une combinaison de diodes 201a qui est spécifique d'une vitesse donnée.

Une fois la vitesse sélectionnée, le microcontrôleur commande l'allumage et l'extinction séquentiels, et en boucle, des diodes 201a, à la vitesse sélectionnée et dans un ordre qui dépend du sens de défilement mémorisé.

Le cas échéant, le praticien peut inverser le sens de défilement, par exemple par un appui prolongé sur le bouton de sélection 202f.

Une fois la règle 20 correctement paramétrée par le praticien (vitesse et sens défilement du repère lumineux RL), le traitement endoveineux peut être commencé.

Après avoir pratiqué une incision dans la peau, le praticien introduit l'extrémité de l'ensemble canule11/fibre otique12 dans la veine à traiter et pousse cet ensemble jusqu'à ce que cette extrémité atteigne le point le plus éloigné de la région de la veine à traiter.

Puis le praticien prend un repère sur la fibre optique (par exemple au moyen de son doigt- figure 4) et tire manuellement sur l'ensemble canule 11/ fibre optique 12, de manière à ce que son repère suive le repère lumineux RL de la règle 20, ce qui permet au praticien de contrôler que la vitesse de retrait correspond à la vitesse de défilement du repère lumineux RL. Pendant ce retrait continu de la fibre optique 12, le praticien actionne de manière usuelle le générateur laser 13 de manière à effectuer un tir laser. La vitesse de retrait continu de la fibre optique 12 étant contrôlée par le praticien au moyen du repère lumineux RL, la dose délivrée (J/cm) lors d'un tir laser est ainsi également contrôlée.

Lorsque le praticien veut effectuer un nouveau tir laser, il doit déplacer la règle 20, le cas échéant la réinitialiser (paramètres vitesse et sens de défilement), ou attendre un tour complet du repère lumineux RL.

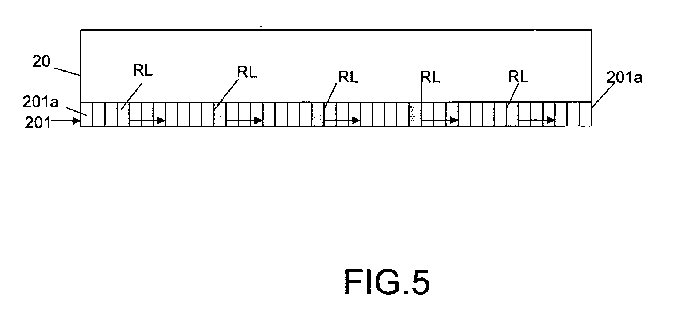

Dans une variante de réalisation illustrée sur la figure 5, la règle 20 comporte plusieurs repères lumineux RL espacés se déplaçant à la même vitesse et dans le même sens, ce qui comparativement à une règle avec repère lumineux unique permet avantageusement au praticien de ne pas devoir attendre q'un repère lumineux atteigne la dernière diode, avant de d'effectuer un tir laser, le praticien se calant sur le repère lumineux le plus proche.

L'invention n'est pas limitée à un dispositif 2 mettant en oeuvre un repère lumineux réalisé à base de diodes. Dans le cas d'un repère lumineux, chaque diode 201 a peut notamment être remplacée par toute autre type de source lumineuse ponctuelle. Dans une autre variante, les sources lumineuses ponctuelles pourraient être remplacées par un écran de type LCD ou autre, le repère visuel étant dans ce cas un curseur affiché sur cet écran et se déplaçant linéairement à la vitesse sélectionnée.

Le repère lumineux RL peut plus généralement être remplacé par tout repère visuel déplacé automatiquement dans une direction donnée à une vitesse prédéfinie. Par exemple, le repère visuel peut être un repère mécanique mobile.

L'invention trouve avantageusement son application au contrôle de la dose délivrée lors du mouvement de retrait continu d'une fibre optique au cours d'un traitement endoveineux, mais n'est toutefois pas limitée à cette seule application.

En particulier, et de manière non exhaustive, l'invention peut être appliquée à tout traitement invasif du corps humain ou animal au moyen de tout type d'instrument qui est apte à être introduit au moins partiellement dans une partie de corps humain ou animal, et qui permet de délivrer localement des doses de traitement dans ladite partie de corps humain ou animal, lors d'un déplacement sensiblement linéaire de l'instrument. L'instrument peut par exemple être une sonde ou aiguille permettant d'appliquer une énergie RF.

La source d'énergie 13 n'est pas nécessairement un générateur de rayonnement électromagnétique, mais pourrait être un générateur RF, une source d'énergie acoustique, par exemple ultrasonore, une source produisant de la vapeur d'eau.

## Revendications

1. Appareil de traitement comportant un instrument invasif (I) qui est apte à être introduit au moins partiellement dans une partie de corps humain ou animal, et qui permet de délivrer localement des doses de traitement dans ladite partie de corps humain ou animal, **caractérisé en ce qu'**il comprend des moyens (2) d'aide au contrôle du déplacement de l'instrument (I) dans la partie de corps humain ou animal, lesquels moyens d'aide au contrôle de déplacement comportent au moins un repère visuel (RL) et des moyens de commande (202) permettant de déplacer automatiquement et linéairement ledit au moins repère visuel (RL) à une vitesse prédéfinie.

2. Appareil selon la revendication 1, **caractérisé en ce que** le repère visuel (RL) est un repère lumineux.

3. Appareil selon la revendication 2, **caractérisé en ce qu'**il comprend une pluralité de sources lumineuses ponctuelles (201 a) alignées, et **en ce que** les moyens de commande (202) sont conçus pour commander l'allumage et l'extinction séquentiels des sources lumineuses (201 a), de manière à créer au moins un repère lumineux (RL) se déplaçant linéairement à une vitesse prédéfinie.

4. Appareil selon la revendication 3, **caractérisé en ce que** les moyens de commande (202) sont conçus pour commander l'allumage et l'extinction séquentiels, et en boucle, desdites sources lumineuses, de manière à créer au moins un repère lumineux (RL) se déplaçant à une vitesse prédéfinie et de manière cyclique entre la première source lumineuses et la dernière source lumineuse.

5. Appareil selon la revendication 3 ou 4, **caractérisé en ce que** les moyens de commande (202) sont conçus pour commander l'allumage et l'extinction séquentiels, et de préférence en boucle, desdites sources lumineuses (201 a), de manière à créer plusieurs repères lumineux (RL) espacés et se déplaçant à la même vitesse prédéfinie.

6. Appareil selon l'une des revendications 1 à 5, **caractérisé en ce que** la vitesse de déplacement du ou des repères visuels (RL) est réglable.

7. Appareil selon l'une des revendications 1 à 6, **caractérisé en ce que** les moyens (2) d'aide au contrôle du déplacement de l'instrument se présentent sous la forme d'une règle (20) apte à être positionnée sur la partie de corps humain ou animal à traiter.

8. Appareil selon l'une des revendications 1 à 7, **caractérisé en ce que** l'instrument (I) est apte à être introduit au moins partiellement dans une veine de manière à permettre la mise en oeuvre d'un traitement endoveineux.

9. Appareil d'un selon l'une des revendications 1 à 8, **caractérisé en ce que** l'instrument (I) comporte une fibre optique (12).

10. Appareil selon l'une des revendications 1 à 8, **caractérisé en ce que** l'instrument (I) comporte un ensemble canule (11)/fibre optique (12) ou un ensemble cathéter/fibre optique.

11. Appareil selon l'une des revendications 1 à 10, **caractérisé en ce qu'**il comporte une source d'énergie (13), et **en ce que** l'instrument est relié à la source d'énergie (13) et est conçu pour appliquer localement l'énergie délivrée par la source.

12. Appareil selon la revendication 11, **caractérisé en ce que** la source (13) est un générateur de rayonnement électromagnétique.

13. Appareil selon la revendication 11, **caractérisé en ce que** la source (13) est une générateur RF.

14. Appareil selon la revendication 11, **caractérisé en ce que** la source (13) est une source d'énergie acoustique

15. Appareil selon la revendication 11, **caractérisé en ce que** la source est une source produisant de la vapeur d'eau.
